(19) Europäisches Patentamt
European Patent Office
Office européen des brevets

(11) Numéro de publication: **0 065 909**
**B1**

(12) **FASCICULE DE BREVET EUROPEEN**

(45) Date de publication du fascicule du brevet: **12.12.84**

(21) Numéro de dépôt: **82400873.4**

(22) Date de dépôt: **11.05.82**

(51) Int. Cl.$^3$: **C 07 D 401/06,**
C 07 D 209/08, A 61 K 31/445

(54) **((Pipéridyl-4)-2 alkyl-1 éthyl)-3 indoles et leur utilisation comme médicaments.**

(30) Priorité: **22.05.81 FR 8110220**

(43) Date de publication de la demande:
**01.12.82 Bulletin 82/48**

(45) Mention de la délivrance du brevet:
**12.12.84 Bulletin 84/50**

(84) Etats contractants désignés:
**BE CH DE FR GB IT LI LU NL SE**

(56) Documents cités:
**EP-A-0 007 258**
**FR-M- 6 291**
**GB-A- 925 429**
**GB-A-1 023 781**

**THE JOURNAL OF ORGANIC CHEMISTRY, vol. 26, no. 7, septembre 1961, A.P. GRAY et al. "The Catalytic Hydrogenation of Indolylylethyl-pyridines. 4-(Indolylethyl)-1-aralkylpiperidines as Potent Analgesics", pages 3368-3373**

(73) Titulaire: **PHARMUKA LABORATOIRES**
**35 Quai du Moulin de Cage**
**F-92231 Gennevilliers (FR)**

(72) Inventeur: **Le Fur, Gérard Roger**
**35, rue du Progrès**
**F-92350 Plessis Robinson (FR)**
Inventeur: **Audiau, François**
**9, rue Guérin**
**F-92350 Plessis-Robinson (FR)**

(74) Mandataire: **Gaumont, Robert et al**
**RHONE-POULENC RECHERCHES Service Brevets Pharma 25, Quai Paul Doumer**
**F-92408 Courbevoie Cedex (FR)**

**EP 0 065 909 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

Le brevet français n° 75 38051 (n° 2 334 358) déposé le 12 Décembre 1975 décrit des dérivés de l'indole, actifs comme inhibiteurs spécifiques de la recapture de la sérotonine par les neurones et pouvant de ce fait trouver des applications comme médicaments psychotropes, plus particulièrement comme antidépresseurs. Comme tels on peut mentionner spécialement le [(pipéridyl-4)-2 éthyl]-3 indole de formule:

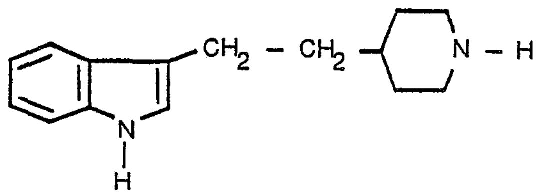

(I)

ou indalpine G. LE FUR et coll. Life Sciences, *23*, 1959 (1978).

La présente invention a pour objet, à titre de produits nouveaux, les composés de formule générale:

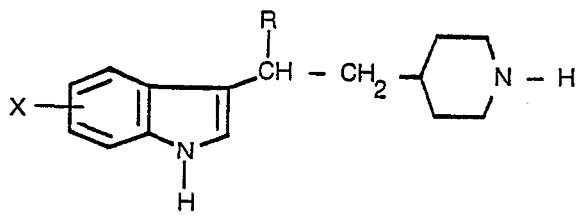

(II)

dans laquelle R représente un groupe alkyle possédant 1 à 3 atomes de carbone et X représente un atome d'hydrogène ou d'halogène, par exemple le fluor ou le chlore.

Il a en effet été trouvé, dans les services de la demanderesse, que les composés de formule (II) possèdent non seulement comme l'indalpine, la propriété d'inhiber la recapture de la sérotonine mais aussi la propriété de provoquer la libération de la sérotonine contenue soit dans les neurones, soit dans les plaquettes sanguines.

Cette double fonction se manifeste par une action plus intense et plus rapide sur la dépression en raison de la libération neuronale de sérotonine dans la fente synaptique, conjuguée à l'inhibition de la recapture. La libération dans le plasma sanguin de la sérotonine plaquettaire améliore les états migraineux; enfin la déplétion en sérotonine des plaquettes prévient la formation de trombi artériels.

Les composés de formule générale (II) peuvent être préparés par action sur les composés de formule générale:

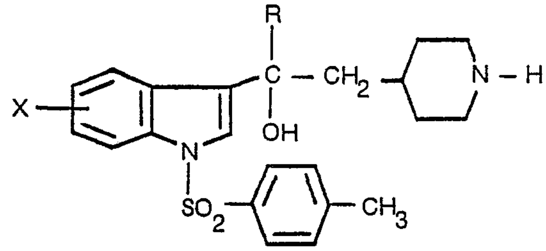

(III)

dans laquelle R et X ont les mêmes significations que dans la formule (II) d'un hydrure métallique, tel que l'hydrure d'aluminium et de lithium, au sein d'un éther, tel que l'éther diéthylique ou le tétra-hydrofuranne, ou d'un mélange d'un éther et d'un hydrocarbure tel que le toluène, à une température comprise entre 0°C et la température d'ébullition du solvant utilisé.

Les matières premières de départ de formule (III) peuvent être préparées suivant le schéma réactionnel ci-dessous:

a) 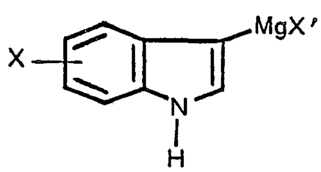 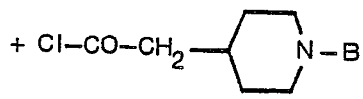

(V)

(IV)

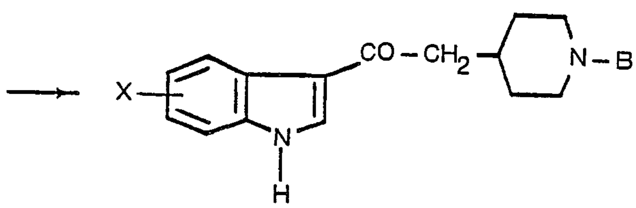

(VI)

b) (VI) + 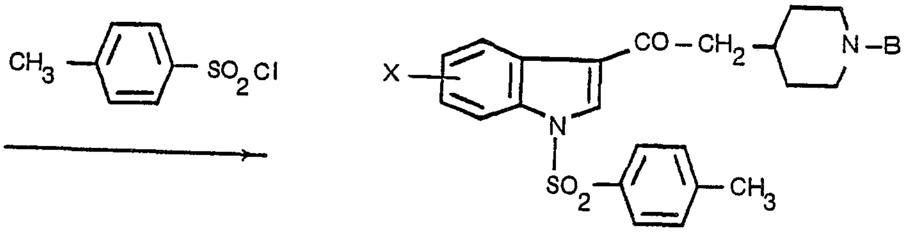

(VII)

c) (VII) 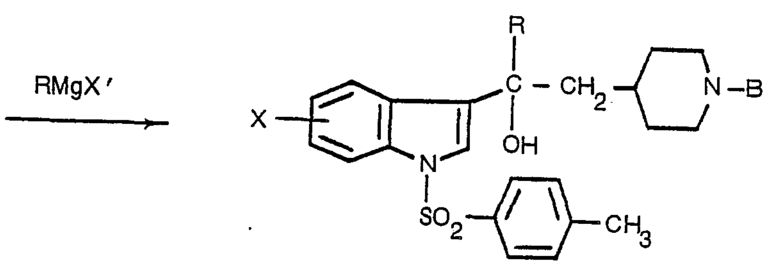


(VIII)

d) (VIII) 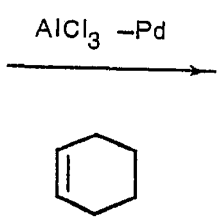


(III)

a) Au réactif de Grignard de formule IV dans laquelle X a les mêmes significations que dans la formule (II) et X' représente un atome de chlore, de brome ou d'iode (obtenu par action sur l'indole correspondant d'un halogénure d'alkylmagnésium tel que le chlorure, le bromure ou l'iodure de méthylmagnésium au sein d'un éther tel que l'éther diéthylique), on ajoute à une température comprise entre −10°C et +20°C le chlorure d'acide de formule (V) (dans laquelle B représente le groupe benzyloxycarbonyle) en solution dans un éther tel que le tétrahydrofuranne ou dans un hydrocarbure tel que le toluène.

b) La cétone de formule (VI) ainsi obtenue est tosylée par action du chlorure de tosyle, à une température comprise entre —20°C et +20°C, au sein d'un solvant inerte et en présence d'une base. On peut par exemple opérer au sein de la pyridine, jouant à la fois le rôle de base et de solvant, ou au sein de l'acétone en présence de carbonate de potassium.

Dans l'étape suivante c) on fait réagir, sur le produit de formule (VII) ainsi obtenu, le réactif de Grignard de formule RMgX' dans laquelle R a la même signification que dans la formule (II) et X' représente un atome de chlore, de brome ou d'iode, au sein d'un solvant inerte tel que l'éther diéthylique ou le tétrahydrofuranne, à une température comprise entre 0° et 30°C.

L'hydrolyse du mélange réactionnel doit être faite à basse température (entre 0 et 10°C) et dans une zone de pH comprise entre 5 et 7.

L'étape d) consiste en la coupure du groupement protecteur B dans des conditions permettant d'éviter la déshydratation de la fonction alcool de la chaîne. On utilise avantageusement à cet effet le cyclohexène en présence de chlorure d'aluminium et de charbon palladié, à la température d'ébullition du cyclohexène.

Le dérivé pipéridinique de formule (V) peut être préparé en 2 étapes à partir de l'acide pipéridyl-4 acétique selon I. DE GRAW, J. Hetero. Chem., *3*, 90 (1966).

Les mélanges réactionnels obtenus suivant les divers procédés décrits précédemment sont traités suivant des méthodes classiques, physiques (évaporation, extraction à l'aide d'un solvant, distillation, cristallisation, chromatographie, etc...) ou chimiques (formation de sel et régénération de la base, etc...) afin d'isoler les composés de formule (II) à l'état pur.

Les composés de formule (II) sous forme de base libre peuvent éventuellement être transformés en sels d'addition avec un acide minéral ou organique par action d'un tel acide au sein d'un solvant approprié.

L'exemple suivant illustre l'invention sand la limiter.

## Exemple 1

[(Piperidyl-4)-2 methyl-1 ethyl]-3 indole

On chauffe pendant 5 heures à 50°C une suspension de 16 g d'hydrure d'aluminium et de lithium, 10 g de (pipéridyl-4)-1 (p.toluènesulfonyloxy-1 indolyl-3)-2 propanol-2 dans 300 ml d'éther et 300 ml toluène.

Après hydrolyse, la phase organique est lavée à l'eau et évaporée sous vide. On obtient 4,8 g d'une huile que l'on purifie par chromatographie sur gel de silice (éluant: toluène-éthanol-diéthylamine: 10—10—1). Après cristallisation dans 6 ml d'acétonitrile on obtient 1,9 g de [(pipéridyl-4)-2 méthyl-1 éthyl]-3 indole fondant à 125°C.

Le produit de départ est préparé de la façon suivante: A une solution d'iodure de méthylmagnésium préparée à partir de 6,5 g de magnésium et de 38,6 g d'iodure de méthyle dans 150 ml d'éther éthylique, on ajoute 17,6 g d'indole en solution dans 100 ml d'éther. On chauffe à reflux 2 heures, puis refroidit vers 0°C avant d'ajouter goutte-à-goutte sous agitation une solution de 36,3 g de chlorure de benzyloxycarbonyl-1 pipéridyl-4 acétyle (brevet ger. Offen. 2 618 152) dans 300 ml de toluène; on agite pendant 2 heures vers 10°C et hydrolyse ensuite avec 170 ml d'acide chlorhydrique 2 N. On obtient 25,8 g de (benzyloxycarbonyl-1 pipéridyl-4)-2 (indolyl-3)-1 éthanone (F: 122°C) que l'on transforme en dérivé tosylé par l'action de 15,7 g de chlorure de tosyle en présence de 27,8 g de carbonate de potassium dans 400 ml d'acétone: on obtient 34,5 g de (benzyloxycarbonyl-1 pipéridyl-4)-2 (p.toluènesulfonyloxy-1 indolyl-3)-1 éthanone que l'on met en suspension dans 600 ml d'éther éthylique. On ajoute peu à peu cette suspension à une solution d'iodure de méthyle magnésium préparée à partir de 3,1 g de magnésium et de 18,5 g d'iodure de méthyle dans 200 ml d'éther éthylique. On agite une nuit à température ambiante et hydrolyse vers 5°C avec 650 ml d'une solution aqueuse à 20% en poids d'acétate d'ammonium. On obtient 35,5 g de (benzyloxycarbonyl-1 pipéridyl-4)-1 (tosyl-1 indolyl-3)-2 propanol-2. Le groupement protecteur de la pipéridine est alors éliminé par l'action du cyclohexène en présence de chlorure d'aluminium et de charbon palladié à 10%; l'addition des réactifs s'effectue vers 0°C sous atmosphère inerte; on termine en chauffant 4 heures à reflux. Après filtration, concentration sous vide et purification par chromatographie sur gel de silice (éluant: toluène-éthanol-diéthylamine-= 5—5—1) on obtient 10 g de (pipéridyl-4)-1 (p.toluènesulfonyloxy-1 indolyl-3)-2 propanol-2, que l'on utilise tel quel pour la préparation du [(pipéridyl-4)-2 méthyl-1 éthyl]-3 indole.

## Proprietes Pharmacologiques

On sait que la recapture de la sérotonine par les plaquettes sanguines constitue un bon modèle de la recapture de cette amine par les neurones (cf. J. TUOMISTO, J. Pharm., Pharmac., *26*, 92 (1974). Appliquée à l'étude des médicaments une méthode mettant en oeuvre les plaquettes sanguines présente le grand intérêt de permettre l'utilisation de cellules humaines, ce qui lui confère un bon caractère prévisionnel.

La capacité des produits à inhiber la recapture de la sérotonine ou à provoquer sa libération a été mise en évidence sur des plaquettes sanguines humaines, selon J. L. DAVID et Coll. "Platelets Function and thrombosis, a review of methods" p. 335 (Plenum Press, LONDON 1972).

1) *Inhibition de la recapture de la sérotonine*

Les résultats sont exprimés par une dose inhibitrice 50% ou $I_{50}$ qui représente la dose de produit, en micromoles par litre, diminuant de 50% la recapture de la sérotonine.

2) *Libération de la sérotonine*

L'action des produits sur la libération de la sérotonine est testée à la concentration de 5 x $10^{-5}$ mole/litre. Les résultats obtenus sont exprimés en pourcentage d'augmentation de la libération de la sérotonine par rapport aux résultats fournis par les témoins.

Les résultats obtenus avec les composés selon l'invention sont rassemblés dans le tableau ci-dessous. Dans ce tableau figure à titre comparatif, les résultats obtenus avec deux produits de référence (imipramine et p-chloroamphétamine) et avec l'indalpine.

TABLEAU

| Produit | Inhibition de la recapture de la sérotonine $I_{50}$ en micromoles par litre | Pourcentage d'augmentation de la libération de la sérotonine (concentration du produit : 5 × $10^{-5}$ mole/litre) |
|---|---|---|
| Exemple 1 | 0,05 | 62 |
| Indalpine | 0,035 | 22 |
| Imipramine | 0,4 | 13 |
| p-chloro-amphétamine | 12 | 51 |

Les produits de l'invention sont donc non seulement de puissants inhibiteurs de la recapture de la sérotonine (activités équivalentes à celle de l'indalpine) mais en outre de puissants agents de libération de la sérotonine, encore plus actifs que la p-chloro-amphétamine.

L'intérêt de ces composés réside dans le fait qu'ils induisent comme la parachloroamphétamine la libération de sérotonine sans pourtant présenter aucune des autres propriétés pharmacologiques caractéristiques des amphétaminiques (anorexie, hypermotilité).

Proprietes toxicologiques

La toxicité aiguë des produits a été déterminée chez la souris mâle $CD_1$ (Charles RIVER) par voie orale. Les composés selon l'invention se comportent comme des substances relativement peu toxiques. A titre d'exemple, le produit de l'exemple 1 est atoxique à la dose de 100 mg/kg chez la souris.

Utilisation therapeutique

Les composés de l'invention et leurs sels pharmaceutiquement acceptables peuvent être utilisés en thérapeutique humaine sous forme de comprimés, capsules, gélules, suppositoires, solutions ingérables ou injectables, etc.. comme réagulateurs du tonus vaculaire sérotonino-dépendant, notamment pour le traitement des migraines, comme agents antithrombosants et comme médicaments thymoanaleptiques à action particulièrement rapide (à cause de leur action sur la libération de la sérotonine).

La posologie dépend des effets recherchés et de la voie d'administration utilisée. Par exemple, par voie orale, elle peut être comprise entre 15 et 250 mg de substance active par jour, avec des doses unitaires allant de 5 à 50 mg.

**Revendications**

1. Les composés de formule générale:

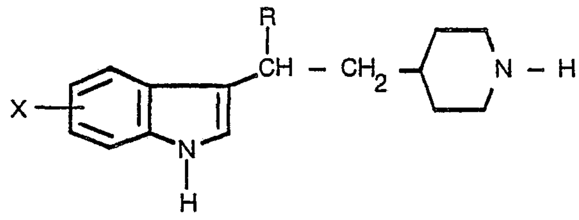

(II)

dans laquelle R représente un groupe alkyle possédant 1 à 3 atomes de carbone et X représente un atome d'hydrogène ou d'halogène.

2. Procédé pour la préparation des composés revendiqués selon la revendication 1 caractérisé en ce que l'on fait réagir sur un composé de formule générale:

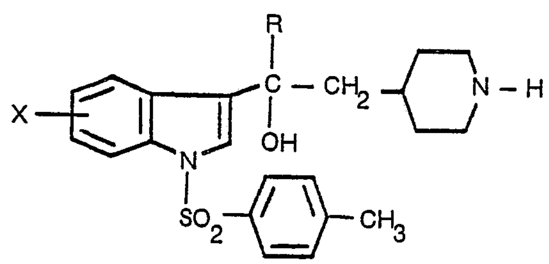

(III)

dans laquelle R et X ont les mêmes significations que dans la formule (II) un hydrure métallique au sein d'un éther ou d'un mélange d'un éther et d'un hydrocarbure, à une température comprise entre 0°C et la température d'ébullition du solvant.

3. En tant qu'agents inhibiteurs de la recapture de la sérotonine et agents libérateurs de la sérotonine, utilisables comme médicaments spécialement comme thymoanaleptiques antimigraineux et antithrombosants, les composés revendiqués selon la revendication 1) et leurs sels avec les acides pharmaceutiquement acceptables.

**Patentansprüche**

1. Die Verbindungen der allgemeinen Formel (II)

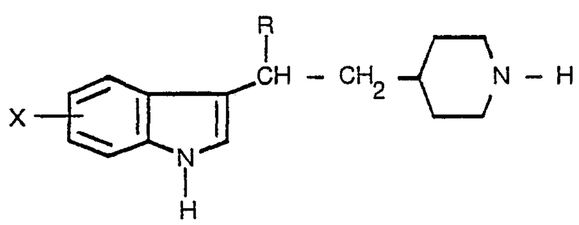

(II)

worin R eine Alkylgruppe mit 1 bis 3 Kohlenstoffatomen und X ein Wasserstoff- oder Halogenatom bedeutet.

2. Verfahren zur Herstellung der gemäß Anspruch 1 beanspruchten Verbindungen, dadurch gekennzeichnet, daß man auf eine Verbindung der allgemeinen Formel (III)

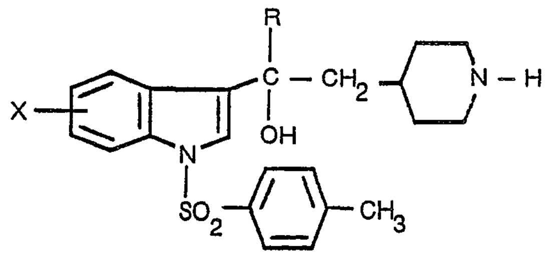

(III)

worin R und X dieselben Bedeutungen wie in Formel (II) haben, ein Metallhydrid in einem Äther oder einem Gemisch eines Äthers und eines Kohlenwasserstoffs bei einer Temperatur zwischen 0°C und der Siedetemperatur des Lösungsmittels einwirken läßt.

3. Als Inhibitoren der Wiederbindung des Serotonins und Mittel zur Freisetzung des Serotonins, die als Arzneimittel, besonders als Thymoanaleptika, Antimigräne- und Antithrombose verwendbar sind, die Verbindungen, die gemäß Anspruch 1 beansprucht sind und ihre Salze mit pharmazeutisch annehmbaren Säuren.

**Claims**

1. The compounds of the general formula

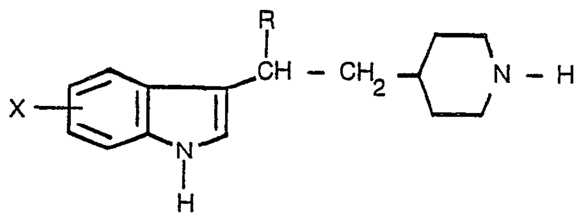

(II)

in which R represents an alkyl group possessing 1 to 3 carbon atoms and X represents a hydrogen atom or halogen atom.

2. Process for the preparation of the compounds claimed according to Claim 1, characterised in that a metal hydride is reacted with a compound of the general formula

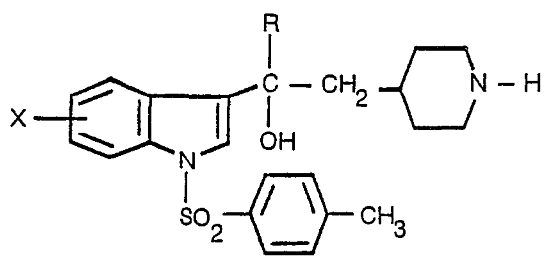

(III)

in which R and X have the same meanings as in formula (II), in an ether or a mixture of an ether and a hydrocarbon, at a temprature of between 0°C and the boiling point of the solvent.

3. As inhibitors of the recapture of serotonine and liberators of serotonine, which may be used as medicaments, especially as thymo-analeptic, anti-migraine and anti-thrombosis agents, the compounds claimed according to Claim 1) and their salts with pharmaceutically acceptable acids.